Europäisches Patentamt

**European Patent Office**

Office européen des brevets

(19)

(11) Publication number: **0 017 314**

**B1**

(12)

# EUROPEAN PATENT SPECIFICATION

(45) Date of publication of patent specification: **20.07.83**

(51) Int. Cl.³: **C 12 M 1/04**

(21) Application number: **80300341.7**

(22) Date of filing: **06.02.80**

(54) Article for formation of anaerobic atmosphere.

(30) Priority: **02.04.79 US 26337**

(43) Date of publication of application:
**15.10.80 Bulletin 80/21**

(45) Publication of the grant of the patent:
**20.07.83 Bulletin 83/29**

(84) Designated Contracting States:
**BE DE FR GB IT NL SE**

(56) References cited:
**DE - A - 2 800 437**
**US - A - 3 246 959**
**US - A - 3 483 089**
**US - A - 3 891 509**
**US - A - 4 038 148**

(73) Proprietor: **Brewer, John H.**
**1402, Woodland Trail**
**Abilene, Texas 79605 (US)**

(72) Inventor: **Brewer, John H.**
**1402, Woodland Trail**
**Abilene, Texas 79605 (US)**

(74) Representative: **Ruffles, Graham Keith et al,**
**MARKS & CLERK 57-60 Lincoln's Inn Fields**
**London WC2A 3LS (GB)**

Courier Press, Leamington Spa, England.

Article for formation of anaerobic atmosphere

This invention relates to gas generation, and more particularly to the formation of anaerobic atmospheres.

Anaerobic atmospheres are required for the culturing of some microorganisms. US Patent No. 3,246,959 discloses equipment for generating an anaerobic atmosphere while US Patent No. 3,483,089 discloses apparatus for anaerobic culturing, with the apparatus making use of a cold platinium catalyst. More specifically, in accordance with US Patent No. 3,246,959, there is provided a package which includes, for example, a material which is capable of generating hydrogen upon being contacted with water. The interior of the package is divided into two compartments, which are in fluid-flow communication with one another. One of the compartments contains the hydrogen-generating material. Upon introduction of water into the other compartment, there is a controlled flow of water into the compartment containing the hydrogen-generating material. The released hydrogen is then reacted with oxygen to give an anaerobic atmosphere, for example by passing an electric current through a platinized wire gauge. Such a package can also be employed in an anaerobic jar as disclosed in US Patent No. 3,483,089.

Another device for generating an anaerobic atmosphere is described in German Offenlegungs-schrift No. 2800437, and is similar to the package of US Patent No. 3246959.

The present invention is directed to an improvement in gas-generating devices.

In accordance with the present invention, there is provided an article for use in forming an anaerobic atmosphere. The article comprises a package, the interior of which includes a substance which is capable of evolving a labile gas which can react with gaseous oxygen, and the exterior of which carries a catalyst for catalyzing reaction between oxygen and the labile gas generated within and released from the package.

Use of the article within a closed environment can lead to reaction with the labile gas of any oxygen in the closed environment, thereby resulting in an anaerobic atmosphere.

In accordance with a preferred embodiment, the substance, preferably in the form of a tablet, is capable of generating hydrogen as the labile gas. A catalyst for oxygen-hydrogen reaction is then applied to the exterior of the package. In accordance with a particularly preferred embodiment, the interior of the package also includes a substance, also preferably in the form of a tablet, which is capable of generating carbon dioxide. In accordance with another preferred aspect of the present invention, the exterior of the package also carries one or more anaerobic indicators for use in showing that anaerobiosis of a proper level has been achieved. The exterior preferably also carries a carbon dioxide indicator for indicating that a required carbon dioxide level has been achieved.

In the present invention, there is provided a carbon dioxide-generating composition. For preference, the composition is formulated in a manner to generate carbon dioxide and provide an acidic pH whereby when used in a gas-generating package along with a hydrogen generator, acidic conditions are maintained thereby to prevent carbon dioxide absorption which might occur under alkaline conditions.

More particularly, the composition of the invention includes a water-soluble solid acid and a water-soluble solid carbonate in amounts suitable for generating carbon dioxide and also providing an acidic pH, in particular, in general, a pH of less than 6 when dissolved in water. As representative examples of suitable acids, there may be mentioned; citric, tartaric, ascorbic, succinic, malic, fumaric or lactic acid. As representative examples of suitable carbonates, there may be mentioned; sodium bicarbonate, sodium carbonate, potassium carbonate, sodium sesquicarbonate, etc. The preferred composition includes citric acid and sodium bicarbonate. The composition is preferably employed in the form of a tablet in which case suitable lubricants and binders are usually also employed. The proportions of acid and carbonate may differ, particularly depending on the materials used. The selection of suitable amounts to provide a desired carbon dioxide concentration for culturing and to provide an acidic pH is within the scope of those skilled in the art. Suitably the composition is formulated for dissolution in from 2 to 10 times its weight of water, e.g. 4 times its weight of water.

The invention will be further described with respect to a preferred embodiment thereof. Such a preferred embodiment is illustrated in the accompanying drawings, wherein:

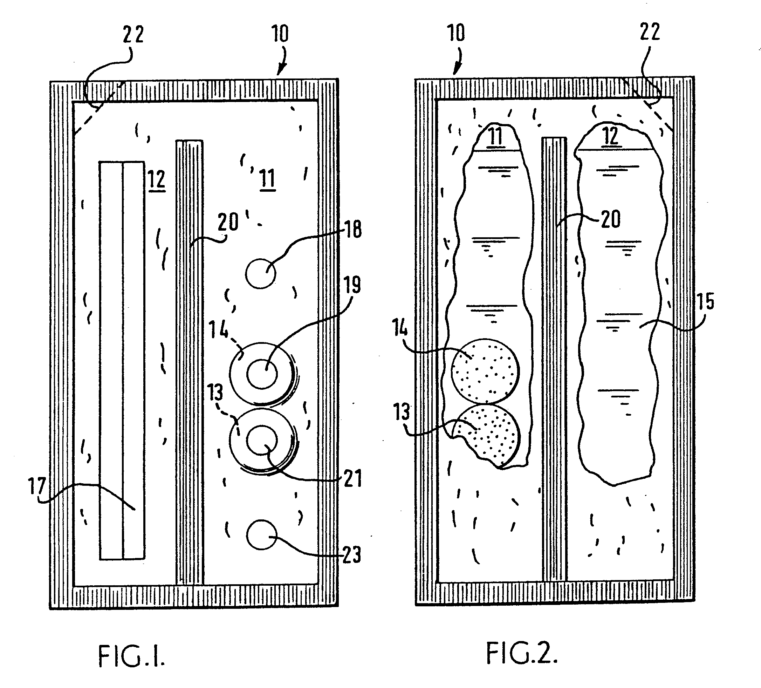

Figure 1 is an elevational view of a preferred embodiment of an article in accordance with the invention;

Figure 2 is a cross-sectional view of the article of Figure 1; and

Figure 3 is an elevational view of an anaerobic jar employing the article of Figures 1 and 2.

Referring now to the drawings, there is shown an article or device for generating an anaerobic atmosphere. The article is a package in the form of an envelope 10 made of a suitable material which is impervious to the atmosphere and moisture, and which is inert to materials contained within the package and gases generated therein and which is not destroyed by the heat of reaction released during use of the package. Thus, for example, the envelope 10 may be formed of a metallic foil, such as aluminium, which is coated on its inner surface with a thermoplastics material, such as polyethylene or a polyvinyl chloride. The envelope 10 may be formed from two panels suitably secured together around the edges by heat sealing. The interior of the envelope is divided into a first gas-generating compartment 11 and a second, liquid-receiving compartment 12 in communication therewith, the division being effected by a suitable partition 20 formed, for example, by heat sealing. The compartment 11 includes gas-generating material in the form of a tablet 13, which includes a substance capable of generating hydrogen, and a tablet 14, which includes a substance capable of generating carbon dioxide. It is to be understood that the number of compartments in the package need not be restricted to two.

The interior compartments 11 and 12 are in internal fluid-flow communication with each other through a fluid transfer means in the form of, for example, filter paper 15 which is capable of permitting both liquid and gas flow between compartments 11 and 12. The filter paper 15 extends through the partition 20 into each of the compartments 11 and 12. As a result of the porosity of the filter paper, liquid and gas can slowly diffuse between the internal compartments. Other porous material such as blotting paper, cotton twill etc, may be employed instead of the filter paper 15. It is also to be understood that although the filter paper is shown as being a single sheet, it may be divided into two or more sheets.

As particularly shown, the partition 20 terminates before the top of the package, whereby the compartments are in communication with each other above the partition. The package is designed to be used in a manner such that liquid introduced into compartment 12 does not reach a level above the partition whereby compartments 11 and 12 are only in gas flow communication above the partition.

In accordance with the present invention, the exterior of package 10 is provided with a catalyst for catalyzing reaction between oxygen in the atmosphere external of the package and the hydrogen generated within and released from the package 10. As particularly shown, the catalyst is a coating 17 on the metallic foil forming the exterior of package 10. It is to be understood that the catalyst need not be applied by coating. Although the catalyst is generally coated on the exterior of the package, other methods of application are possible; for example, by providing the exterior of the package with an adhesive and dusting on the catalyst. In coating or otherwise applying the catalyst to the package exterior, the application should be effected in a manner such that the other materials employed, such as coating materials, do not mask the catalyst. Particular means for applying the catalyst to the package exterior can readily be chosen to suit particular requirements.

The catalyst is applied to the exterior of the package in an amount sufficient for catalyzing the reaction between hydrogen and oxygen thereby to generate an anaerobic atmosphere. The catalyst can be comprised of palladium or platinum, supported on a suitable support, such as carbon, aluminium oxide or other metal oxide, which is then coated or otherwise applied onto the exterior of the package.

Although the catalyst is generally shown in the form of stripes, it is to be understood that the catalyst may be applied in another form. The catalyst may be applied to the package in a wide variety of forms and in a wide variety of locations. In some cases, if the catalyst is applied as a concentrated spot over the tablet, scorching of the package might occur, which should be avoided to prevent possible contamination. The specific arrangement of the catalyst to achieve satisfactory results presents no difficulties to the skilled man.

In accordance with a particularly preferred embodiment, an anaerobic indicator is also applied to the exterior of package 10. Such an indicator may be coated or otherwise applied to the metallic foil, and such indicator, as particularly shown, can be in the form of three different indicator spots 18, 19 and 21. The indicators may be of for example methylene blue, resazurin, and indigo carmine. Such compounds have different oxidation-reduction potentials and respond to different levels of oxygen: the use of such indicators will indicate different levels of oxygen. A similar indicator for monitoring the level of carbon dioxide is also applied to the external surface of the metallic foil as spot 23. Although such indicators have been particularly shown as being printed as spots on the exterior of the package, it is to be understood that the indicators may take other forms such as lettering or other designs.

As should be apparent, the package forms an integral unit for providing an anaerobic atmosphere in that the package includes the materials for generating hydrogen and carbon dioxide, as well as the catalyst for catalyzing the reaction between hydrogen and oxygen and the indicators for indicating anaerobic conditions and carbon dioxide concentration.

In employing the gas generating device of the present invention, the liquid receiving compartment 12 is opened by cutting or tearing away a corner of the package 10 along a tear line 22. A material, such as water, capable of interacting with the gas-generating substances in compartment 11 is introduced into compartment 12, and flows into compartment 11, at a controlled rate, through the filter paper 15. Upon contact with the water, the tablets 13 and 14 generate hydrogen and carbon dioxide, which flow from compartment 11 into compartment 12 through the filter paper 15 and/or through the open upper channel, and ultimately into the container in which the envelope has been placed. The hydrogen reacts with oxygen in the container, with the reaction being catalyzed by the catalyst 17 applied to the exterior of package 10. In addition, anaerobic conditions are indicated by means of the indicators 18, 19 and 21 which are also applied to the exterior of package 10. Carbon dioxide concentration is indicated by indicator 23 which is also applied to the exterior of the package.

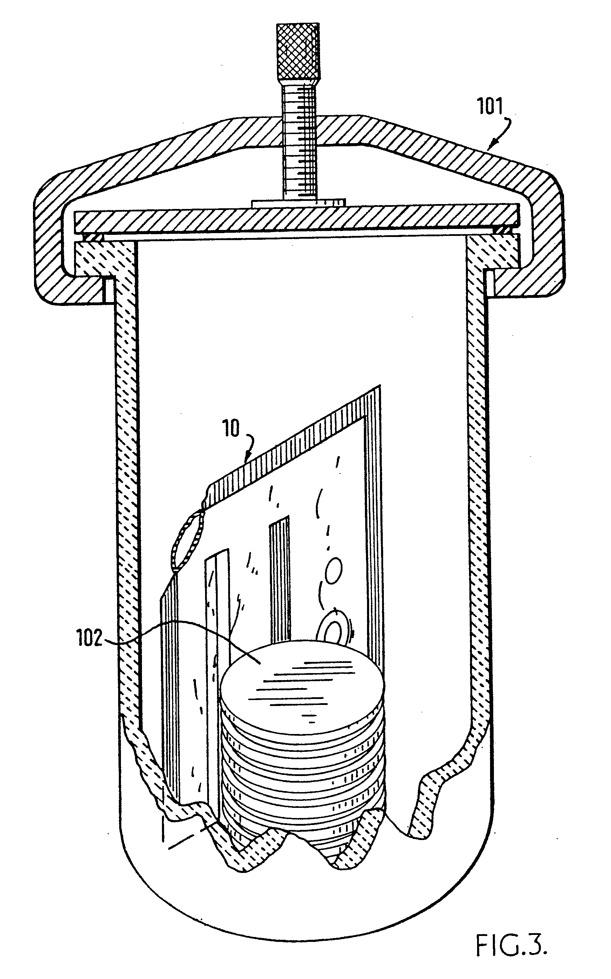

Thus, for example, as shown in Figure 3 the gas-generating package 10 is employed in an anaerobic jar 101 including culture plates 102. The jar does not include a cold catalyst for the oxygen-hydrogen reaction nor a separate indicator.

The invention will be yet further described with resepct to the following example:

Example
The following exemplified the components and construction of a package in accordance with the present invention. The package is of aluminium foil, coated on its inner surface, and includes a carbon dioxide-generating tablet, a hydrogen-generating tablet, a catalyst for the reaction between hydrogen and oxygen applied to the exterior of the package, the anaerobic and carbon dioxide indicators applied to the exterior of the package.

Carbon dioxide generating tablet
1.85           citric acid
0.73—0.82      sodium bicarbonate
In addition, the tablet includes suitable binder and lubricant: eg. 0.1 g boric acid and 0.015 g magnesium stearate.

Hydrogen generating tablet
0.9 g sodium borohydride
The tablet further includes suitable binder and lubricant.
Optionally, the tablet can be coated with a water-soluble gelatin to prevent decomposition.

Formulation of catalyst coating
0.3333 g of gum of tragacanth
1.2 g of 5% carbon palladium catalyst
The ingredients are boiled, cooled and then shaken for uniformity.

Formulation of indicator base
18 g fructose
1.53 g $K_2HPO_4$
0.35 g NaOH
2.5 mg phenylmercuric nitrate

Formulation of methylene blue indicator
indicator base
5 mg methylene blue
1.2 g of the dry indicator mixture with 0.3333 g, gum of tragacanth is added to 40 ml of water and boiled.
After cooling it is ready for use.

Formulation of indigo carmine indicator
indicator base
5 ml indigo carmine
1.2 g of dry indicator mixture with 0.3425 g gum of tragacanth is mixed with 40 ml of water and boiled. After cooling it is ready for use.

Formulation of resazurin indicator
indicator base
5 mg of resazurin
1.2 g of dry indicator mixture and 0.3345 g gum of tragacanth is added to 40 ml of water and boiled. After cooling it is ready for use.

Formulation of carbon dioxide indicator
0.02 g sodium bicarbonate
0.001 g bromo thymol blue
0.3333 g gum of tragacanth
The components are mixed with 40.0 ml of water and boiled. After cooling, the mixture is ready for use.

Application of cataylst
Sufficient catalyst coating is applied as a stripe to the foil exterior to provide at least 0.001 g of palladium catalyst, and the stripe dried.

Application of indicator
methylene blue—1 drop (0.05 ml) of indicator is applied to the exterior of aluminium surface of the package and dried.
indigo carmine—1 drop (0.05 ml) of indicator is applied to the exterior aluminium surface of the package and dried.
resazurin—1 drop (0.05 ml) of indicator is applied to the exterior aluminium surface of the package and dried.

The package in accordance with the example is employed as a gas generator, with such gas generation being effected by the addition of 10 ml of water to the liquid-receiving compartment.

Although the invention has been described with respect to a preferred embodiment thereof, it is to be understood that modifications are possible. Thus, for example, a liquid other than water itself could be employed for generating the gas, and tablets other than those particularly described could also be employed for generating gas. Similarly, the tablet could be formulated for producing hydrogen in a manner other than as particularly described; eg, hydrogen could be generated by another liquid, such as an acid; in particular, hydrochloric acid, although the use of water itself is preferred. Similarly, the liquid for generating the gas upon contacting the tablet could be within the package in a separate compartment or ampoule. Thus, the present invention is not limited to the particularly described embodiment: it is readily possible to adopt configurations or constructions other than the ones particularly described.

The present invention is particularly advantageous in that it is no longer necessary to employ a specific type of container or jar for generating anaerobic conditions. The gas generating package is self-contained; ie the package contains the material for generating the gas, as well as catalyst and optionally indicator. Moreover, the present invention allows provision of fresh catalyst each time a run is made, thereby eliminating the previous problem of catalyst poisoning from a previous run. Moreover, heat of reaction from the tablet or other gas-generating substance is transferred through the package and activates the catalyst more rapidly than cold catalyst in the lid of the jar, as heretofore employed in the art. Simultaneously, heat generated by the catalyst increases the rate of reaction for the substance. Furthermore, in accordance with a preferred embodiment, anaerobic indicator is provided on the package thereby eliminating the necessity of adding a separate indicator.

In addition, the carbon dioxide generating compositions employed in accordance with the present invention can be formulated to produce an acid pH during gas generating within the package and thereby inhibit carbon dioxide absorption which could occur under alkaline conditions. In addition, the tablets as described are capable of providing an atmosphere of 7.5 to 8.5% carbon dioxide even after 24 hours.

## Claims

1. An article for generating an anaerobic atmosphere for use in culturing anaerobic microorganisms, the article comprising a flexible sealed package (10), a substance (13) in the package which when contacted with water generates hydrogen and a substance (14) in the package which is capable of generating carbon dioxide, characterized in that a catalyst (17) is applied to the exterior surface of the flexible sealed package, whereby when the package is broken and water contacts the hydrogen-generating substance, the externally-coated applied catalyst catalyzes the reaction between oxygen and the generated hydrogen.

2. An article according to claim 1, wherein the flexible sealed package is of a metallic foil.

3. An article according to claim 1 or 2, wherein at least one anaerobic indicator (18, 19, 21) is applied to the exterior of the flexible sealed package.

4. An article according to any preceding claim, wherein at least one carbon dioxide indicator (23) is applied to the exterior of the package.

5. An article according to any preceding claim, wherein the flexible sealed package has first and second interior compartments (11, 12), with the first interior compartment containing the substances for generating hydrogen and carbon dioxide and the second compartment having a removable portion to facilitate opening of the package for introduction of water, and there being a porous fluid transfer means (15) allowing liquid transfer from the second compartment to the first compartment.

6. An article according to any preceding claim, wherein the carbon dioxide-generating substance (14) is a water-soluble solid carbonate and a water-soluble solid acid.

7. An article according to any preceding claim, wherein the hydrogen-generating substance (13) is sodium borohydride.

8. An article according to any preceding claim, wherein the catalyst (17) is supported palladium or supported platinum.

9. An article according to claim 8, wherein the catalyst is a carbon platinum catalyst coated on the exterior of the package using gum of tragacanth.

10. An article according to claim 9, wherein sufficient catalyst is applied to provide at least 0.001 g of the palladium catalyst on the package exterior.

## Patentansprüche

1. Artikel zum Bilden einer anaeroben Atmosphäre zur Anwendung bei der Kultivation anaerober Mikroorganismen, bestehend aus einer flexiblen, versiegelten Packung (10), einer darin befindlichen Substanz (13), die beim Kontakt mit Wasser Wasserstoff erzeugt, und aus einer darin befindlichen, zur Erzeugung von Kohlendioxid geeigneten Substanz (14), dadurch gekennzeichnet, daß ein Katalysator (17) an der äußeren Oberfläche der flexiblen, versiegelten Packung angebracht ist und daß, wenn die Packung aufgebrochen wird und Wasser mit der wasserstofferzeugenden Substanz in Kontakt kommt, der außen angebrachte Katalysator die Reaktion zwischen Sauerstoff und dem erzeugten Wasserstoff katalysiert.

2. Artikel nach Anspruch 1, dadurch gekennzeichnet, daß die flexible, versiegelte Packung aus einer metallischen Folie besteht.

3. Artikel nach Anspruch 1 oder 2, dadurch gekennzeichnet, daß zumindest ein anaerober Indikator (18, 19, 21) an der Außenseite der flexiblen, versiegelten Packung angebracht ist.

4. Artikel nach einem der vorhergehenden Ansprüche, dadurch gekennziechnet, daß zumindest ein Kohlendioxid-Indikator (23) an der Außenseite der Packung angebracht ist.

5. Artikel nach einem der vorhergehenden Ansprüche, dadurch gekennzeichnet, daß die flexible, versiegelte Packung erste und zweite innere Abteile (11, 12) aufweist, wobei das erste innere Abteil die Substanzen zur Erzeugung von Wasserstoff und Kohlendioxid, das zweite Abteil einen entfernbaren Abschnitt aufweist, um die Packung zu öffnen und Wasser einzuführen, und daß poröse Mittel (15) zur Fluid-Übertragung vorgesehen sind, die den Flüssigkeitstransport vom zweiten Abteil in das erste Abteil gestatten.

6. Artikel nach einem der vorhergehenden Ansprüche, dadurch gekennzeichnet, daß die Kohlendioxid erzeugende Substanz (14) ein wasserlösliches, festes Carbonat und eine wasserlösliche, feste Säure ist.

7. Artikel nach einem der vorhergehenden Ansprüche, dadurch gekennzeichnet, daß die Wasserstoff erzeugende Substanz (13) Natriumborhydrid ist.

8. Artikel nach einem der vorhergehenden Ansprüche, dadurch gekennzeichnet, daß der Katalysator (17) auf einem Träger angeordnetes Palladium oder auf einem Träger angeordnetes Platin ist.

9. Artikel nach Anspruch 8, dadurch gekennzeichnet, daß der Katalysator ein Kohlenstoff-Platin-Katalysator ist, der mittels Gummi aus Traganth auf der Außenseite der Packung aufgetragen ist.

10. Artikel nach Anspruch 9, dadurch gekennzeichnet, daß eine genügende Katalysatormenge auf der Packungsaußenseite angebracht ist, die zumindest 0,001 g Palladiumkatalysator zur Verfügung stellt.

## Revendications

1. Article pour établir une atmosphère anaérobie en vue de la culture de micro-organismes anaérobies, l'article comprenant un paquet hermétique flexible (10), une substance (13) dans le paquet qui, lorsqu'on la met en contact avec l'eau, engendre de l'hydrogène et une substance (14) dans le paquet qui est capable d'engendrer de l'anhydride carbonique, caractérisé en ce qu'un catalyseur (17) est appliqué à la surface extérieure du paquet hermétique flexible, de sorte que lorsqu'on brise le paquet et que l'eau vient en contact avec la substance génératrice d'hydrogène, le catalyseur appliqué par revêtement externe catalyse la réaction entre l'oxygène et l'hydrogène engendré.

2. Article selon la revendication 1, dans lequel le paquet hermétique flexible est en clinquant métallique.

3. Article selon la revendication 1 ou 2, dans lequel au moins un indicateur anaérobie (18, 19, 21) est appliqué sur l'extérieur du paquet hermétique flexible.

4. Article selon l'une quelconque des revendications précédentes, dans lequel au moins un indicateur (23) d'anhydride carbonique est appliqué à l'extérieur du paquet.

5. Article selon l'une quelconque des revendications précédentes, dans lequel le paquet hermétique flexible comprend un premier et un second compartiments intérieurs (11, 12), le premier compartiment intérieur contenant les substances pour engendrer l'hydrogène et l'anhydride carbonique et le second compartiment présentant une partie amovible pour faciliter l'ouverture du paquet en vue d'introduction d'eau, des moyens poreux (15) de transfert de fluide permettant le transfert du liquide du second compartiment au premier compartiment.

6. Article selon l'une quelconque des revendications précédentes, dans lequel la substance (14) génératrice d'anhydride carbonique est un carbonate solide soluble dans l'eau et un acide solide soluble dans l'eau.

7. Article selon l'une quelconque des revendications précédentes, dans lequel la substance (13) génératrice d'hydrogène est le borohydrure de sodium.

8. Article selon l'une quelconque des revendications précédentes, dans lequel le catalyseur (17) est le palladium sur support ou le platine sur support.

9. Article selon la revendication 8, dans lequel le catalyseur est un catalyseur de platine sur carbone revêtant l'extérieur du paquet avec utilisation de gomme adragante.

10. Article selon la revendication 9, dans lequel on applique une quantité suffisante de catalyseur pour fournir au moins 0,001 g de catalyseur au palladium sur l'extérieur du paquet.

**0017314**

FIG.I.

FIG.2.

FIG.3.